# EUROPEAN PATENT APPLICATION

(11) **EP 4 636 070 A1**
(43) Date of publication of application: **22.10.2025**
(21) Application number: 23903865.6
(22) Date of filing: 04.12.2023
(51) Int. Cl.: C12M 3/00, C12M 1/34, C12M 1/00, C12M 3/06

(54) **DECELLULARIZATION REACTOR**

(30) Priority: 16.12.2022 KR 20220177589
(71) Applicant: POSCO Holdings Inc., Pohang-si, Gyeongsangbuk-do 37859 (KR); RESEARCH INSTITUTE OF INDUSTRIAL SCIENCE & TECHNOLOGY, Pohang-si, Gyeongsangbuk-do 37673 (KR); POSTECH Research and Business Development Foundation, Pohang-si, Gyeongsangbuk-do 37673 (KR)
(72) Inventor: LEE, Boram, Busan 48113 (KR)
(74) Representative: Pfenning, Meinig & Partner mbB
(86) International application number: PCT/KR2023/019821
(87) International publication number: WO 2024/128662

(57) **Abstract**

The decellularization reaction apparatus is provided. The decellularization reaction apparatus according to the present invention comprises a first reaction vessel for storing a first decellularization solution for performing a decellularization reaction, a first ex-situ analysis application device for extracting a tissue of a first xenogeneic organ and a first decellularization solution undergoing a decellularization reaction within the first reaction vessel and measuring the physical property of the first decellularization solution ex-situ.

## Description

### [Technical Field]

The present invention relates to a decellularization reaction apparatus, and more particularly, to a decellularization reaction apparatus for processing small quantities of various types of xenogeneic organ.

### [Background Art]

Recently, with the global trend of aging populations, the number of patients with chronic diseases is increasing, and interest in artificial organ manufacturing technology using 3D bioprinting is growing.

Bio-ink is a term referring to ink material for printing artificial organs with a 3D bio printer and is manufactured based on extracellular matrix obtained after decellularizing xenogeneic organs.

If the amount of xenogeneic organ to be decellularized is large, a reactor type suitable for mass production may be used. However, if the amount of xenogeneic organ tissue that may be supplied, such as the cornea of a pig, is small, for example, about 10 g, it is difficult to operate a large-scale facility.

In addition, there is an urgent need for a device that may decellularize a small amount and wide variety of xenogeneic organ tissue, such as the detailed tissues of the heart (left atrium, left ventricle, right atrium, and right ventricle), and at the same time compare the differences in physical property between tissue of xenogeneic organ.

### [DETAILED DESCRIPTION OF THE INVENTION]

### [Technical Problem]

The present invention provides a decellularization reaction device capable of efficiently decellularizing small quantities of various types of xenogeneic organ tissues.

### [Technical Solution]

A decellularization reaction apparatus according to an embodiment of the present invention may comprise a first reaction vessel for storing a first decellularization solution to perform decellularization on the tissue of a first xenogeneic organ to be decellularized inside.

Additionally, the decellularization reaction device may comprise one or more first ex-situ analysis application devices for extracting the first decellularization solution, which is undergoing a decellularization reaction with the tissue of the first xenogeneic organ designated for decellularization within the first reaction vessel, to the outside of the first reaction vessel and for measuring a physical property of the first decellularization solution ex-situ.

The first ex-situ analysis application device may comprise a first mesh chamber inserted into the first reaction vessel and configured to accommodate the first xenogeneic organ tissue to be decellularized.

The first ex-situ analysis application device may comprise a first solution circulation part connected in a way that allows communication between the first reaction vessel and a first cover that covers the first reaction vessel and intended to circulate a first decellularization solution to the first reaction vessel and the first cover.

Additionally, the first ex-situ analysis application device may comprise a first solution sampling part connected to the first solution circulation part, which is configured to extract and sample the first decellularization solution from the first solution circulation part to the outside.

The first lifting part may be combined with the first cover and allows the first cover to be lifted vertically.

The first mesh chamber may comprise a first driving part for rotating the first mesh chamber to be stirred within the first reaction vessel.

The first mesh chamber may be formed in a cylindrical shape with an empty interior.

The outer surface of the first mesh chamber may be arranged in a lattice form with a first mesh.

The first lid for covering the first mesh chamber may be detachably coupled to the upper part of the first mesh chamber.

The first reaction vessel may be manufactured from a transparent material or may have a transparent window, enabling observation of the first reaction vessel interior from the outside.

The first reaction vessel may have a double jacket structure consisting of a first inner tube and a first outer tube.

The first outer tube may be equipped with a first refrigerant injection port for injecting refrigerant into the space between the first outer tube and the first inner tube, and a first refrigerant discharge port for discharging the injected refrigerant to the outside of the first outer tube.

The first reaction vessel may have a first solution outlet for discharging the decellularized solution to the outside of the first reaction vessel.

The first cover may be equipped with a first injection port for injecting the first decellularization solution into the interior of the first reaction vessel.

The first cover may be equipped with a first level sensor to measure the level of the first decellularized solution within the first reaction vessel.

The first driving part may comprise a first rotational shaft coupled with the first lid of the first mesh chamber, a first driving motor installed on the first cover of the first reaction vessel and coupled with the first rotational shaft to rotate the first rotational shaft.

The first solution circulation part may comprise a first circulation tube connected between the first circulation port of the first cover and a first solution outlet, to circulate the first decellularization solution within the first reaction vessel, and a first transfer pump installed on the first circulation tube to transfer the first decellularization solution discharged from the first solution outlet to the first circulation port.

The first circulation tube may be made of expandable the flexible tube.

The first solution sampling part may be connected to the first circulation tube and may comprise a first solution sampling tube for extracting and sampling a first decellularization solution circulated in the first circulation tube.

The physical property of the first decellularization solution may be analyzed by measuring the absorbance (UV-Vis), electrical conductivity, and turbidity of the first decellularization solution sampled from the first solution sampling tube.

The first lifting part may comprise a first coupling plate coupled to the first cover to be vertically movable, and a first lifting cylinder having a first lifting rod, the first lifting cylinder being coupled to one end of the first coupling plate to raise and lower the first coupling plate.

A decellularization reaction device according to another embodiment of the present invention may comprise a first reaction vessel for storing a first decellularization solution to perform decellularization on the tissue of a first xenogeneic organ to be decellularized inside.

In addition, a decellularization reaction device according to another embodiment may comprise one or more first ex-situ analysis application devices for extracting the first decellularization solution, which is undergoing a decellularization reaction with a tissue of the first xenogeneic organ designated for decellularization within the first reaction vessel, to the outside of the first reaction vessel, and for measuring a physical property of the first decellularization solution through ex-situ.

In addition, a decellularization reaction device according to another embodiment may comprise a second reaction vessel for storing a second decellularization solution to perform decellularization on the tissue of a second xenogeneic organ to be decellularized inside.

A decellularization reaction apparatus according to another embodiment may comprise one or more second ex-situ analysis application devices arranged in parallel with the first ex-situ analysis application device and configured to extract the second decellularization solution, which is undergoing a decellularization reaction with the tissue of the second xenogeneic organ within the second reaction vessel, to the outside of the second reaction vessel and measure a physical property of the second decellularization solution ex-situ.

The second xenogeneic organ may be the same as or different from the first xenogeneic organ and the second decellularization solution may be the same as or different from the first decellularization solution

The first ex-situ analysis application device may comprise a first mesh chamber inserted into the first reaction vessel and configured to accommodate the first xenogeneic organ tissue to be decellularized, and a first solution circulation part connected in a way that allows communication between the first reaction vessel and a first cover that covers the first reaction vessel and intended to circulate a first decellularization solution to the first reaction vessel and the first cover.

Additionally, the first ex-situ analysis application device may comprise a first solution sampling part connected to the first solution circulation part, which is configured to extract and sample the first decellularization solution from the first solution circulation part to the outside.

The first ex-situ analysis application device may comprise a first lifting part combined with the first cover and allows the first cover to be lifted vertically, and a first driving part coupled to the first mesh chamber and configured to rotate the first mesh chamber to facilitate agitation within the first reaction vessel.

And, the second first ex-situ analysis application device may comprise a second mesh chamber inserted within the second reaction vessel and configured to accommodate the second xenogeneic organ tissue to be decellularized.

The second ex-situ analysis application device may comprise a second solution circulation part connected in a way that allows communication between the second reaction vessel and a second cover that covers the second reaction vessel and intended to circulate a second decellularization solution to the second reaction vessel and the second cover.

Additionally, the second ex-situ analysis application device may comprise a second solution sampling part connected to the second solution circulation part, which is configured to extract and sample the second decellularization solution from the second solution circulation part to the outside.

The second lifting part may be combined with the second cover and allows the second cover to be lifted vertically.

The second mesh chamber may comprise a second driving part for rotating the second mesh chamber to be stirred within the second reaction vessel.

The second mesh chamber may be formed in a cylindrical shape with an empty interior.

The outer surface of the second mesh chamber may be arranged in a lattice form with a second mesh.

The second lid for covering the second mesh chamber may be detachably coupled to the upper part of the second mesh chamber.

The second reaction vessel may be manufactured from a transparent material or may have a transparent window, enabling observation of the second reaction vessel interior from the outside.

The second reaction vessel may have a double jacket structure consisting of a second inner tube and a second outer tube.

The second outer tube may be equipped with a second refrigerant injection port for injecting refrigerant into the space between the second outer tube and the second inner tube, and a second refrigerant discharge port for discharging the injected refrigerant to the outside of the second outer tube.

The second reaction vessel may have a second solution outlet for discharging the decellularized solution to the outside of the second reaction vessel.

The second cover may be equipped with a second injection port for injecting the second decellularization solution into the interior of the second reaction vessel.

The second driving part may comprise a second rotational shaft coupled with the second lid of the second mesh chamber, a second driving motor installed on the second cover of the second reaction vessel and coupled with the second rotational shaft to rotate the second rotational shaft.

The second solution circulation part may comprise a second circulation tube connected between the second cover and a second solution outlet, to circulate the second decellularization solution within the second reaction vessel, and a second transfer pump installed on the second circulation tube to transfer the second decellularization solution discharged from the second solution outlet to the second circulation port.

The second circulation tube may be made of expandable the flexible tube.

The second solution sampling part may be connected to the second circulation tube and may comprise a second solution sampling tube for extracting and sampling a second decellularization solution circulated in the second circulation tube.

The physical property of the second decellularization solution may be analyzed by measuring the absorbance (UV-Vis), electrical conductivity, and turbidity of the second decellularization solution sampled from the second solution sampling tube.

The second lifting part may comprise a second coupling plate coupled to the second cover to be vertically movable, and a second lifting cylinder having a second lifting rod, the second lifting cylinder being coupled to one end of the second coupling plate to raise and lower the second coupling plate.

### [Advantageous Effects]

According to an embodiment of the present invention, the decellularization process for small quantities of various types of xenogeneic organs may be operated very effectively by utilizing the twin system of the first and second reaction vessels, the first and second mesh chambers, the circulation structure of the first and second decellularization solutions, and the first and second ex-situ analysis application devices, and the physical property of each tissue may be evaluated very efficiently.

### [Description of the Drawings]

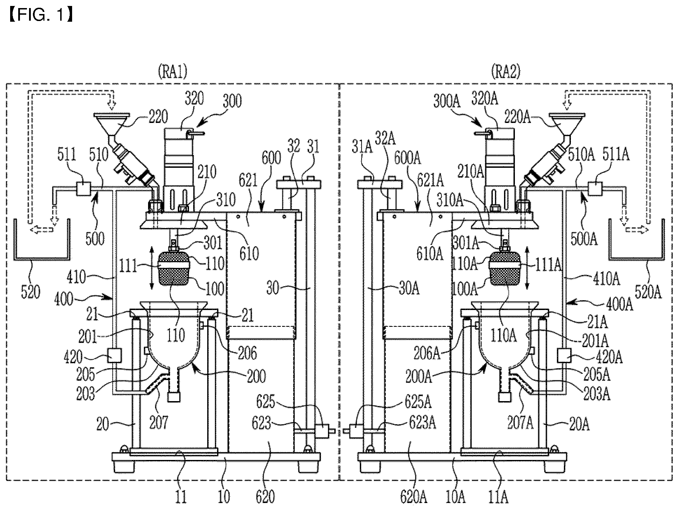

FIG. 1 is a schematic configuration diagram of a decellularization reaction apparatus according to one embodiment of the present invention, showing the first and second covers in an open state.

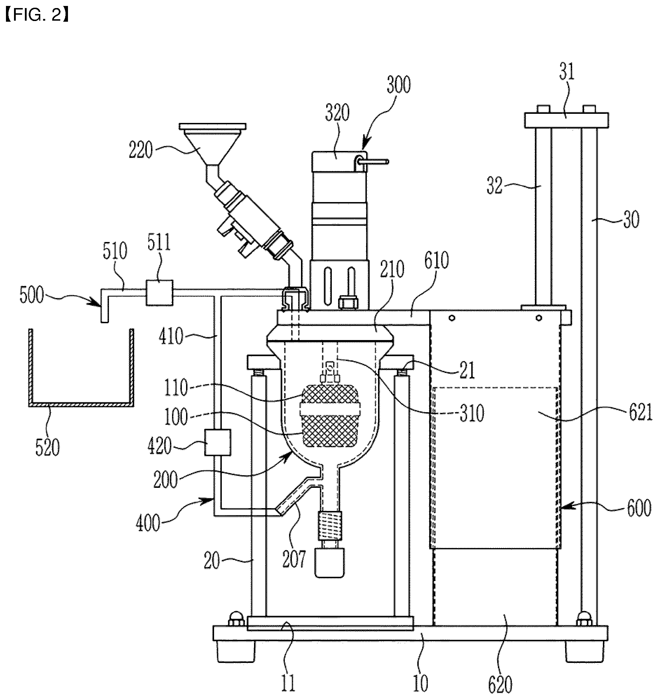

FIG. 2 is a configuration diagram of the first ex-situ analysis application device of the decellularization reaction apparatus according to one embodiment of the present invention, with the first cover closed.

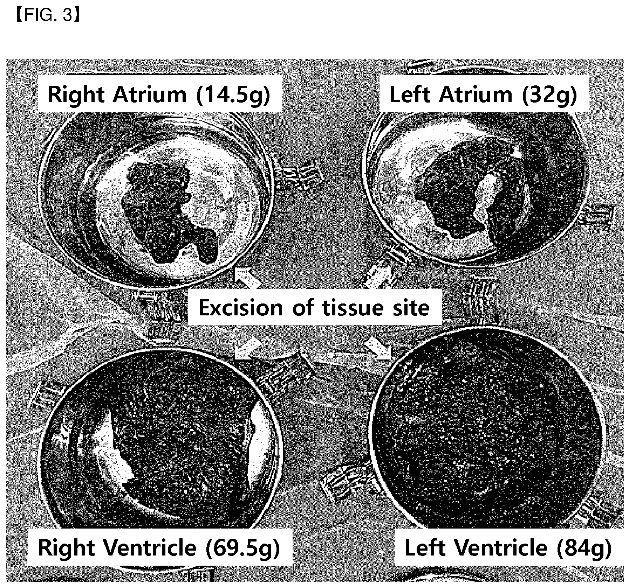

FIG. 3 is a photograph showing the pretreatment process for each part of a pig heart for use in a decellularization reaction device according to one embodiment of the present invention.

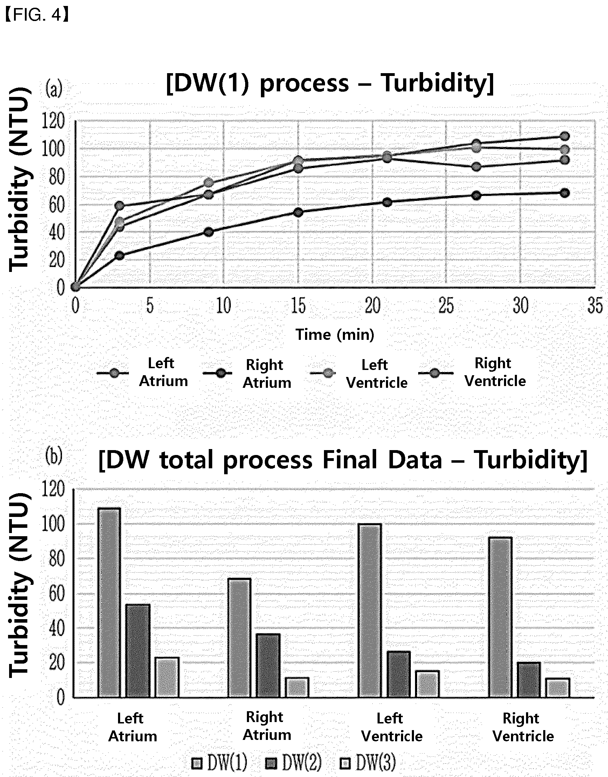

FIG. 4 is a graph showing the result of turbidity analysis over time in the first ultrapure water process (DW(1)) during the decellularization process for each tissue of pig heart muscle using a decellularization reaction device according to one embodiment of the present invention, and the results of turbidity analysis of the first decellularization solution using three ultrapure water processes [DW(1)-DW(2)-DW(3)].

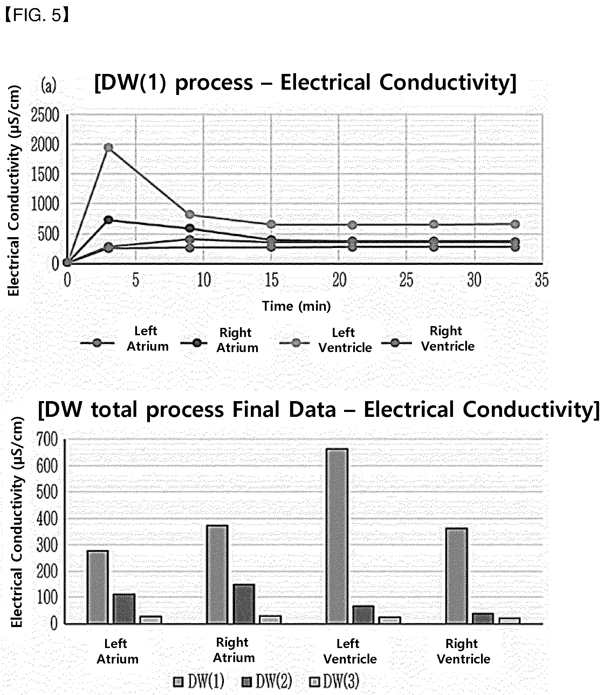

FIG. 5 is a graph showing the result of an electrical conductivity analysis over time in the first ultrapure water process (DW(1)) during the decellularization process for each tissue of a pig heart using a decellularization reaction device according to one embodiment of the present invention, and the results of an electrical conductivity analysis of the first decellularization solution using three ultrapure water processes [DW(1)-DW(2)-DW(3)].

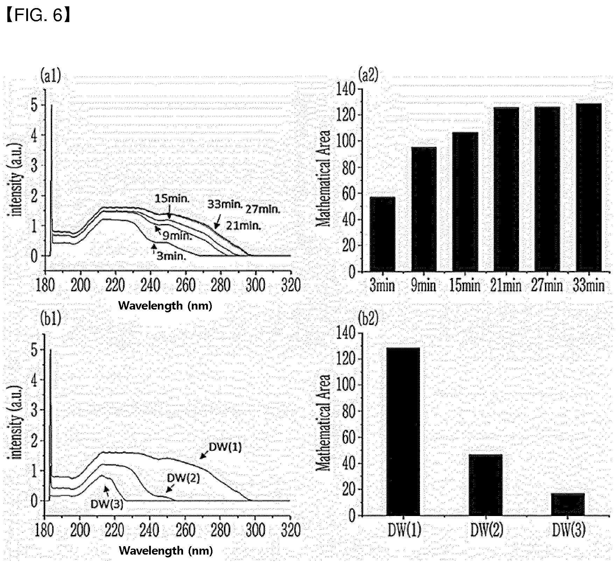

FIG. 6 is a graph showing the result of absorbance analysis over time in the first ultrapure water process (DW(1)) during the decellularization process for each tissue of pig heart muscle using a decellularization reaction device according to one embodiment of the present invention, and the results of absorbance analysis of the first decellularization solution of the left ventricle using three ultrapure water processes [DW(1)-DW(2)-DW(3)].

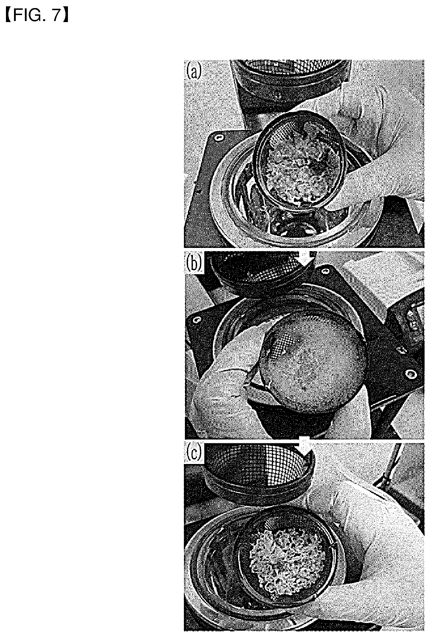

FIG. 7 is a photograph showing the decellularization process of heart muscle tissue using the first decellularization solution, in which ultrapure water and various organic solvents are utilized, as the decellularization reaction progresses in stages (a)-(b)-(c) with the decellularization reaction apparatus according to an embodiment of the present invention.

### [BEST MODE FOR INVENTION]

Hereinafter, with reference to the attached drawings, an embodiment of the present invention will be described so that a person having ordinary skill in the art to which the present invention pertains can easily implement the invention. As can be easily understood by a person of ordinary skill in the technical field to which the present invention pertains, the embodiments described later may be modified in various forms without departing from the concept and scope of the present invention. As much as possible, identical or similar parts are represented in the drawing using the same drawing symbol.

The technical terms used below are merely for the purpose of mentioning specific embodiments and are not intended to limit the present invention. The singular forms used here comprise plural forms unless the phrases clearly indicate the opposite meaning. The meaning of ' comprising' used in the specification specifies certain characteristics, areas, integers, stages, actions, elements, and/or components, and does not exclude the presence or addition of other specific characteristics, areas, integers, stages, actions, elements, components, and/or groups.

All terms including technical and scientific terms used below have the same meaning as understood generally by a person of ordinary skill in the technical field to which the invention pertains. The terms defined in the dictionary are interpreted as having meanings that correspond to the relevant technical literature and the currently disclosed content, and unless otherwise defined, are not interpreted in an ideal or very formal sense.

FIG. 1 is a schematic configuration diagram of a decellularization reaction apparatus according to one embodiment of the present invention, showing the first and second covers in an open state.

FIG. 2 is a configuration diagram of the first ex-situ analysis application device of the decellularization reaction apparatus according to one embodiment of the present invention, with the first cover closed.

Referring to FIG. 1 and 2, the decellularization reaction apparatus according to the first embodiment of the present invention may comprise a first ex-situ analysis application device RA1 and a second ex-situ analysis application device RA2.

The first ex-situ analysis application device RA1 is provided with at least one or more and is capable of externally extracting the first decellularization solution, which is undergoing a decellularization reaction with the tissue of the first xenograft organ to be decellularized, allowing for the analysis of various property of the first decellularization solution ex-situ.

The second ex-situ analysis application device RA2 is arranged at least one side by side with the first ex-situ analysis application device RA1 at a set distance, and is capable of externally extracting the second decellularization solution, which is undergoing a decellularization reaction with the tissue of the second xenograft organ to be decellularized, allowing for the analysis of various property of the second decellularization solution ex-situ.

Here, ex-situ analysis may refer to extracting the first decellularization solution and the second decellularization solution externally from the first ex-situ analysis application device RA1 and the second ex-situ analysis application device RA2 and measuring and analyzing respectively the physical property of the first decellularization solution and the second decellularization solution.

The first xenogeneic organ may be the same as or different from the second xenogeneic organ, and the first decellularization solution may be the same as or different from the second decellularization solution.

In addition, the first ex-situ analysis application device RA1 may comprise a first mesh chamber 100, a first reaction vessel 200, a first driving part 300, a first solution circulation part 400, and a first solution sampling part 500.

The first mesh chamber 100 has a set size and shape and may accommodate the tissue of the first xenogeneic organ to be decellularized inside.

In addition, the first reaction vessel 200 may store a first decellularization solution for performing a decellularization reaction of the tissue of the first heterologous organ in the first mesh chamber 100, which is inserted inside the first mesh chamber 100.

The first driving part 300 may be coupled with the first mesh chamber 100 and may be rotated to stir the first mesh chamber 100 within the first reaction vessel 200.

The first solution circulation part 400 may be connected to each other in a way that communicates between the first reaction vessel 200 and the first cover 210 that covers the first reaction vessel 200, and may circulate the first decellularization solution to the first reaction vessel 200 and the first cover 210.

The first solution sampling part 500 may be connected to the first solution circulation part 400 and, in order to measure various physical property of the first decellularization solution, may extract and sample the first decellularization solution from the first solution circulation part 400 to the outside.

The first cover 210 may be placed on the upper part of the first reaction vessel 200 to cover the first reaction vessel 200.

In addition, the first cover 210 may be coupled or separated from the first reaction vessel 200 so that the first cover 210 may be lifted vertically (in the Y direction of FIG. 1) with respect to the first reaction vessel 200 for the purpose of a first lifting part 600 being able to be coupled.

The first mesh chamber 100 may be easily rotated by the first driving part 300 and may be made in a cylindrical shape that is empty inside to accommodate the tissue of the first xenogeneic organ to be decellularized, etc.

In addition, when the first mesh chamber 100 is made in a square shape, the tissue of the xenogeneic organ may become caught or remain at the angled corners when the first mesh chamber 100 rotates, and the tissue of the xenogeneic organ may not be stirred uniformly. Therefore, the first mesh chamber 100 is made in a cylindrical shape, etc., to stir the tissue of the xenogeneic organ uniformly and efficiently.

The first mesh chamber 100 may be configured as a small type capable of accommodating up to less than 25g of the first xenogeneic organ tissue to be decellularized.

The outer surface of the first mesh chamber 100 is arranged in a lattice form with the first mesh 101, and the first mesh 101 may have a set size, for example, a size of about 1 mm, for a decellularization reaction of tissue of the first xenogeneic organ accommodated in the first mesh chamber 100.

In addition, the size of the first mesh 101 may be set to a size slightly smaller than the tissue of the first xenogeneic organ so that the tissue of the first xenogeneic organ accommodated in the first mesh chamber 100 does not escape out of the first mesh 101 during the decellularization process.

The decellularization reaction apparatus may comprise a first lid 110 for covering the first mesh chamber 100, and which is detachably connected to the upper part of the first mesh chamber 100.

The first lid 110 may have a shape such as a buckle 111 so that it may be easily coupled to the first mesh chamber 100, but it is not limited to this, and it is of course possible for the first lid 110 and the first mesh chamber 100 to be coupled by means of screw coupling, etc.

The upper part of the first lid 110 may be coupled with the first driving part 300.

The first mesh chamber 100 may be manufactured or coated with a material having good chemical resistance, such as Teflon, depending on the property of the first decellularization solution.

Additionally, the first reaction vessel 200 may be manufactured from a transparent material or may have a transparent window (not shown) to visually observation of the decellularization process inside the first reaction vessel 200 from the outside.

The first reaction vessel 200 may be arranged at a predetermined interval on the first base 10 and may be supported by the 1-1 support frame 20 arranged in a vertical direction (Y direction in FIG. 1) with respect to the first base 10.

The first reaction vessel 200 may be made of a material that is resistant to corrosion for the decellularization solution to be used.

In addition, the first reaction vessel 200 may have a double jacket structure consisting of a first inner tube 201 and a first outer tube 203, to control the temperature of the decellularization process.

The first outer tube 203 may be equipped with a first refrigerant injection port 205 for injecting refrigerant into the space between the first outer tube 203 and the first inner tube 201, and a first refrigerant discharge port 206 for discharging the injected refrigerant to the outside of the first outer tube 203.

The first reaction vessel 200 may have a first solution outlet 207 for discharging the decellularization solution to the outside of the first reaction vessel 200.

The first cover 210 may be made in the form of an O-ring made of rubber material or the like so as to seal the first reaction vessel 200 while cushioning impact when lowered by the first lifting part 600 and coming into contact with the upper portion of the first reaction vessel 200.

The first cover 210 may be equipped with a first injection port (not shown) to which a first solution injection tube 220 is connected for injecting a first decellularization solution into the interior of the first reaction vessel 200.

The first decellularization solution may be composed of, for example, ultrapure water, an organic solvent, etc.

Additionally, the first cover 210 is connected to the first solution circulation part 400 to allow the first decellularization solution circulated in the first solution circulation part 400 to be injected into the interior of the first reaction vessel 200.

A first level sensor 213 for measuring the level of the first decellularization solution in the first reaction vessel 200 may be installed in the first cover 210, and the first level sensor 213 may be operated by ultrasonic means, etc.

The first driving part 300 may comprise a first rotation shaft 310 and a first driving motor 320.

The first rotational shaft 310 may be coupled with the first lid 110 of the first mesh chamber 100.

Additionally, the first driving motor 320 may be installed on the first cover 210 of the first reaction vessel 200 and is coupled to the first rotational shaft 310 to enable the rotation of the first rotational shaft 310.

The first drive motor 320 may be made of a magnetic type of motor, etc., to easily rotate the first rotational shaft 310.

The first rotational shaft 310 may be positioned in the center of the upper part of the first lid 110 in a vertical direction (Y direction in FIG. 2) relative to the upper part of the first lid 110 and may be coupled with the first lid 110 through fastening with the first bolt 301 and so on.

The first rotational shaft 310 may be rotated clockwise or counterclockwise by the first driving motor 320, thereby rotating the first mesh chamber 100 to stir the tissue of the first xenogeneic organ accommodated in the first mesh chamber 100, which may be facilitate the decellularization by the first decellularization solution injected into the first reaction vessel 200.

When the first mesh chamber 100 may be rotated at a set rotation speed (e.g., 1 to 500 rpm) to stir, the stirring may be performed alternately in the forward (clockwise) and reverse (counterclockwise) directions to prevent overload of the first driving motor 320 and to prevent entanglement between xenogeneic organs, and a stop time may be set in the middle.

The first solution circulation part (400) may comprise a first circulation tube (410) and a first transfer pump (420).

The first circulation tube 410 is connected between the first circulation port (not shown) arranged in the first cover 210 and the first solution outlet 207 and may circulate the first decellularization solution within the first reaction vessel 200.

Additionally, the first transfer pump 420 may be installed in the first circulation tube 410 and may quantitatively transfer the first decellularization solution flowing out from the first solution outlet 207 to the first circulation port.

The first circulation tube 410 may be formed of a flexible tube to allow for easy expansion and contraction when the first cover 210 is lifted by the first lifting part 600.

In addition, the first circulation tube 410 is connected to a first waste liquid discharge tube 430 for discharging the waste liquid of the first decellularization solution, and a first waste liquid discharge tube control valve 431 may be installed in the first waste liquid discharge tube 430 to control the discharge of the first decellularization solution.

The first solution sampling part 500 may be connected to the first circulation tube 410 and may comprise a first solution sampling tube 510 for extracting and sampling the first decellularization solution circulated in the first circulation tube 410.

After the first decellularization solution sampled from the first solution sampling tube 510 may be contained in a first small container 520 such as a vial, various physical property of the first decellularization solution contained in the first small container 520, such as absorbance (UV-Vis), electrical conductivity, and turbidity, may be measured and analyzed.

The first solution sampling tube 510 may be equipped with a first solution sampling tube control valve 511 to control the opening and closing of the first solution sampling tube 510.

And, the first lifting part 600 may comprise the first coupling plate 610 and the first lifting cylinder 620.

The first coupling plate 610 may be coupled to the first cover 210 so as to be able to be lifted as one piece.

Additionally, the first lifting cylinder 620 is coupled to the lower end of the first coupling plate 610 and may have a first lifting rod 621 that elevates the first coupling plate 610.

The first lifting cylinder 620 may be installed and supported on the upper surface of the first base 10 in a vertical direction (Y direction in FIG. 1) relative to the first base 10.

The first lifting cylinder 620 is connected to the first air supply tube 623 for supplying air into the first lifting cylinder 620, and a first regulator 625 may be installed in the first air supply tube 623 for adjusting the pressure of the air supplied to the first air supply tube 623.

Additionally, the first base 10 may be arranged parallel to the 1-1 support frame 20, and the 1-2 support frame 30 supported by the first base may be installed.

The 1-2 support frame 30 may be connected by a first connecting frame 31 and may be coupled with a first guide rod 630 for guiding the lifting of the first lifting rod 621.

Additionally, the first reaction vessel 200 and the 1-1 support frame 20 may be configured to be movable and detachable for ease of cleaning, and a first groove 11 may be arranged on the upper surface of the first base 10 to align the positions of the first reaction vessel 200 and the first cover 210.

A first elastic member 21 may be coupled with the upper part of the 1-1 support frame 20 to absorb the impact generated when the first cover 210 descends to seal the first reaction vessel 200.

And, the second ex-situ analysis application device RA2 may comprise a second mesh chamber 100A, a second reaction vessel 200A, a second driving part 300A, a second solution circulation part 400A, and a second solution sampling part 500A.

The second mesh chamber (100A) has a set size and shape and may accommodate the tissue of the second xenogeneic organ to be decellularized inside.

Additionally, the second reaction vessel (200A) is arranged parallel to at least one or more with a set distance from the first reaction vessel (200A), and may store a second decellularization solution for performing a decellularization reaction with the tissue of the second xenogeneic organ within the second mesh chamber (100A) inserted therein.

The second driving part (300A) may be coupled with the second mesh chamber (100A) and may be rotated to allow the second mesh chamber (100A) to be stirred within the second reaction vessel (200A).

The second solution circulation part 400A is connected to each other in a way that communicates between the second reaction vessel 200A and the second cover 210A that covers the second reaction vessel 200A, and may circulate the second decellularization solution to the second reaction vessel 200A and the second cover 210A.

The second solution sampling part 500A is connected to the second solution circulation part 400A and, in order to measure various physical property of the second decellularization solution, may extract and sample the first decellularization solution from the second solution circulation part 400A to the outside.

The second cover 210A may be placed on the upper part of the second reaction vessel 200A to cover the second reaction vessel 200A.

In addition, the second cover 210A may be coupled or separated from the second reaction vessel 200A so that the second cover 210A may be lifted vertically (in the Y direction of FIG. 1) with respect to the second reaction vessel 200A for the purpose of a second lifting part 600A being able to be coupled.

The second mesh chamber 100A may be easily rotated by the second driving part 300A and may be made in a cylindrical shape that is empty inside to accommodate the tissue of the second xenogeneic organ to be decellularized, etc.

In addition, when the second mesh chamber 100A is made in a square shape, the tissue of the xenogeneic organ may become caught or remain at the angled corners when the second mesh chamber 100A rotates, and the tissue of the xenogeneic organ may not be stirred uniformly. Therefore, the second mesh chamber 100A is made in a cylindrical shape, etc., to stir the tissue of the xenogeneic organ uniformly and efficiently.

The outer surface of the second mesh chamber 100A is arranged in a lattice form with the second mesh 101A, and the second mesh 101A may have a set size, for example, a size of about 1 mm, for a decellularization reaction of tissue of the second xenogeneic organ accommodated in the second mesh chamber 100A.

The second mesh chamber 100A may be configured as a small type capable of accommodating up to less than 25g of the second xenogeneic organ tissue to be decellularized.

In addition, the size of the second mesh 101A may be set to a size slightly smaller than the tissue of the second xenogeneic organ so that the tissue of the second xenogeneic organ accommodated in the second mesh chamber 100A does not escape out of the second mesh 101A during the decellularization process.

The decellularization reaction apparatus may comprise a second lid 110A for covering the second mesh chamber 100A, and which is detachably connected to the upper part of the second mesh chamber 100A.

The second lid 110A may have a shape such as a buckle 111A so that it may be easily coupled to the second mesh chamber 100A, but it is not limited to this, and it is of course possible for the second lid 110A and the second mesh chamber 100A to be coupled by means of screw coupling, etc.

The upper part of the second lid 110A may be coupled with the second driving part 300A.

The second mesh chamber 100A may be manufactured or coated with a material having good chemical resistance, such as Teflon, depending on the property of the second decellularization solution.

Additionally, the second reaction vessel 200A may be manufactured from a transparent material or may have a transparent window (not shown) to visually observation of the decellularization process inside the second reaction vessel 200A from the outside.

The second reaction vessel 200A may be arranged at a predetermined interval on the second base 10A and may be supported by the 2-1 support frame 20A arranged in a vertical direction (Y direction in FIG. 1) with respect to the second base 10A.

The second reaction vessel 200A may be made of a material that is resistant to corrosion for the decellularization solution to be used.

In addition, the second reaction vessel 200A may have a double jacket structure consisting of a second inner tube 201A and a second outer tube 203A, to control the temperature of the decellularization process.

The second outer tube 203A may be equipped with a second refrigerant injection port 205A for injecting refrigerant into the space between the second outer tube 203A and the second inner tube 201A, and a second refrigerant discharge port 206A for discharging the injected refrigerant to the outside of the second outer tube 203A.

The second reaction vessel 200A may have a second solution outlet 207A for discharging the decellularization solution to the outside of the second reaction vessel 200A.

The second cover 210A may be made in the form of an O-ring made of rubber material or the like so as to seal the second reaction vessel 200A while cushioning impact when lowered by the second lifting part 600A and coming into contact with the upper portion of the second reaction vessel 200A.

The second cover 210A may be equipped with a second injection port (not shown) to which a second solution injection tube 220A is connected for injecting a second decellularization solution into the interior of the second reaction vessel 200A.

The second decellularization solution may be composed of, for example, ultrapure water, an organic solvent, etc.

Additionally, the second cover 210A is connected to the second solution circulation part 400A to allow the second decellularization solution circulated in the second solution circulation part 400A to be injected into the interior of the second reaction vessel 200A.

A second level sensor 213A for measuring the level of the second decellularization solution in the second reaction vessel 200A may be installed in the second cover 210A, and the second level sensor 213A may be operated by ultrasonic means, etc.

The second driving part 300A may comprise a second rotation shaft 310A and a second driving motor 320A.

The second rotational shaft 310A may be coupled with the second lid 110A of the second mesh chamber 100A.

Additionally, the second driving motor 320A may be installed in the second cover 210A of the second reaction vessel 200A and may be coupled to the second rotational shaft 310A to enable the rotation the second rotational shaft 310A.

The second drive motor 320 A may be made of a magnetic type of motor, etc., to easily rotate the second rotational shaft 310 A.

The second rotational shaft 310A may be positioned in the center of the upper part of the second lid 110A in a vertical direction (Y direction in FIG. 1) relative to the upper part of the second lid 110A and may be coupled with the second lid 110A through fastening with the second bolt 301A and so on.

The second rotational shaft 310A may be rotated clockwise or counterclockwise by the second driving motor 320A, thereby rotating the second mesh chamber 100A to stir the tissue of the second xenogeneic organ accommodated in the second mesh chamber 100A, which may facilitate the decellularization by the second decellularization solution injected into the second reaction vessel 200A.

When the second mesh chamber 100A may be rotated at a set rotation speed (e.g., 1 to 300 rpm) to stir, the stirring may be performed alternately in the forward (clockwise) and reverse (counterclockwise) directions to prevent overload of the second driving motor 320A and to prevent entanglement between xenogeneic organs, and a stop time may be set in the middle.

The second solution circulation part 400A may comprise a second circulation tube 410A and a second transfer pump 420A.

The second circulation tube 410A may be connected between the second circulation port (not shown) arranged in the second cover 210A and the second solution outlet 207A and may circulate the second decellularization solution within the second reaction vessel 200A.

Additionally, the second transfer pump 420A may be installed in the second circulation tube 410A and may quantitatively transfer the second decellularization solution flowing out from the second solution outlet 207A to the second circulation port.

The second circulation tube 410A may be formed of a flexible tube to allow for easy expansion and contraction when the second cover 210A may be lifted by the second lifting part 600A.

In addition, the second circulation tube 410A may be connected to a second waste liquid discharge tube 430A for discharging the waste liquid of the second decellularization solution, and a second waste liquid discharge tube control valve 431A may be installed in the second waste liquid discharge tube 430A to control the discharge of the second decellularization solution.

The second solution sampling part 500A may be connected to the first circulation tube 410A and may comprise a second solution sampling tube 510A for extracting and sampling the second decellularization solution circulated in the second circulation tube 410A.

After the second decellularization solution sampled from the second solution sampling tube 510A may be contained in a second small container 520A such as a vial, various physical property of the second decellularization solution contained in the second small container 520A, such as absorbance (UV-Vis), electrical conductivity, and turbidity, may be measured and analyzed.

The second solution sampling tube 510A may be equipped with a second solution sampling tube control valve 511A to control the opening and closing of the second solution sampling tube 510A.

And, the second lifting part 600A may comprise the second coupling plate 610A and the second lifting cylinder 620A.

The second coupling plate 610A may be coupled to the second cover 210A so as to be able to be lifted as one piece.

Additionally, the second lifting cylinder 620A may be coupled to the lower end of the second coupling plate 610A and may have a second lifting rod 621A that elevates the second coupling plate 610A.

The second lifting cylinder 620A may be installed and supported on the upper surface of the second base 10A in a vertical direction (Y direction in FIG. 1) relative to the second base 10A.

The second lifting cylinder 620A may be connected to the second air supply tube 623A for supplying air into the second lifting cylinder 620A, and a second regulator 625 may be installed in the second air supply tube 623A for adjusting the pressure of air supplied to the second air supply tube 623A.

Additionally, the second base 10A may be arranged parallel to the 2-1 support frame 20A, and the 2-2 support frame 30A supported by the second base may be installed.

The 2-2 support frame 30A may be connected by a second connecting frame 31 and may be coupled with a second guide rod 630A for guiding the lifting of the second lifting rod 621A.

Additionally, the second reaction vessel 200A and the 2-1 support frame 20A may be configured to be movable and detachable for ease of cleaning, and a second groove 11A may be arranged on the upper surface of the second base 10A to align the positions of the second reaction vessel 200A and the second cover 210A.

A second elastic member 21A may be coupled with the upper part of the 2-1 support frame 20A to absorb the impact generated when the second cover 210A descends to seal the second reaction vessel 200A.

Hereinafter, with reference to FIGS. 1 and 2, the operation of the decellularization reaction apparatus according to one embodiment of the present invention will be described.

First, the tissue of the first xenogeneic organ to be decellularized and the first decellularization solution for the decellularization reaction with the tissue of the first xenogeneic organ may be prepared, and the tissue of the second xenogeneic organ to be decellularized and the second decellularization solution for the decellularization reaction with the tissue of the second xenogeneic organ may be prepared.

Here, an example is provided in which the first xenogeneic organ and the second xenogeneic organ are different, and the first decellularization solution and the second decellularization solution are also different, however, the same applies in cases when the first xenogeneic organ and the second xenogeneic organ are identical, or when the first decellularization solution and the second decellularization solution are the same.

Hereinafter, an example will be provided in which the first xenogeneic organ tissue and the first decellularization solution are injected into the first mesh chamber 100 and the first reaction vessel 200 using the first ex-situ analysis application device RA1, however, the same applies when the second xenogeneic organ tissue and the second decellularization solution are injected into the second mesh chamber 100A and the second reaction vessel 200A.

In order to inject the tissue of the first xenogeneic to be decellularized and the first decellularization solution into the first mesh chamber 100 and the first reaction vessel 200, respectively, the first lid 110 must be opened from the first mesh chamber 100, and the first cover 210 must be in an open state from the first reaction vessel 200, as shown in FIG. 1.

In this state, the tissue of the first xenogeneic to be decellularized may be inserted into the interior of the first mesh chamber 100, and after injecting the first decellularization solution into the first reaction vessel 200, the upper part of the first mesh chamber 100 is covered with the first lid 110, and then the first lid 110 is attached to the first mesh chamber 100 using a buckle 111.

And, when the first lifting rod (621) of the first lifting cylinder (620) of the first lifting part (600) is operated to contract, as shown in FIG. 2, as the first lifting rod (621) is lowered to a set length, and the first cover (210) covers the upper part of the first reaction vessel (200) to seal the first reaction vessel (200).

As such, when the first cover 210 seals the first reaction vessel 200, the first mesh chamber 100 is inserted into the interior of the first reaction vessel 200, as shown in FIG. 2.

In addition, while the above describes the case of directly injecting the first decellularization solution into the first reaction vessel 200, it is not limited thereto, and as another method for injecting the first decellularization solution, the first decellularization solution may be injected into the first reaction vessel 200 through a first injection port (not shown) to which the first solution injection tube 220 is connected.

And, when the injection of the first decellularization solution into the first reaction vessel 200 is completed, the first driving motor 320 of the first driving part 300 is driven to rotate the first rotation shaft 310 clockwise or counterclockwise at a set speed to rotate the first mesh chamber 100.

In this way, as the first mesh chamber 100 is rotated and stirred, the tissue of the first xenogeneic organ in the first mesh chamber is stirred and undergoes a decellularization reaction with the first decellularization solution in the first reaction vessel 200.

At this time, the tissue of the first xenogeneic organ is stirred by the rotation of the first mesh chamber 100 and the first decellularization solution is vertically moved from the first cover 210 into the first reaction vessel 200 through the first circulation tube 410, so that the tissue of the first xenogeneic organ and the first decellularization solution are well mixed, thereby obtaining a first decellularization solution with a homogeneous concentration in real time.

In this state, the first transfer pump 420 is operated to circulate the first decellularization solution, which is undergoing decellularization reaction in the first reaction vessel 200, through the first circulation tube 410 at a set speed.

The first decellularization solution circulated from the first solution outlet 207 of the first reaction vessel 200 to the first cover 210 through the first circulation tube 410 may be extracted and sampled through the first solution sampling tube 510 by controlling the first solution sampling pipe control valve 511 to the open state.

In this way, the first decellularization solution sampled from the first solution sampling tube 510 is placed in the first small container 520 such as a vial, various physical property of the first decellularized solution contained in the first small container 520, such as absorbance (UV-Vis), electrical conductivity, and turbidity, may be measured and analyzed.

In addition, by sampling and measuring the first decellularization solution of a homogeneous concentration, various physical property of the first decellularization solution, such as absorbance (UV-Vis), electrical conductivity, and turbidity, may be easily analyzed, enabling accurate understanding of the progress of the decellularization process.

### (embodiment)

Hereinafter, as an example of the first xenogeneic to be decellularized, a decellularization experiment for each part of the pig heart (left atrium/left ventricle/right atrium/right ventricle) will be described.
1) As shown in Figure 3, the pig heart is cut into tissues by each part (left atrium, left ventricle, right atrium, and right ventricle) and followed by a preprocessing procedure that comprises the removal of blood vessels and foreign substances.
2) As shown in Fig. 3, each part of the tissue (maximum 25 g) is placed into the first mesh chamber in the first reaction vessel, and then the first lid of the first mesh chamber is closed.
   The tissues of each part of the pig heart is partially cut using scissors or similar tools, and If the tissues of each part of the pig heart are cut to a size smaller than the first mesh size of the first mesh chamber, they may escape and be lost outside the first mesh during the decellularization process and be lost, so caution should be taken.
3) In the case of cardiac muscle, the decellularization solution injected into the first reaction vessel is various, such as primary ultrapure water, SDS aqueous solution, Triton-X aqueous solution, EtOH, and PBS aqueous solution, And considering the volume of the first reaction vessel and the immersion height of the first mesh chamber, the injection amount of each solution is, for example, 600 ml.
   First, primary ultrapure water is injected into each of the first reaction vessels to remove foreign substances and decellularize the heart muscle.
4) After that, the gas (air) is pressurized into the first lifting cylinder to move the first cover at the upper portion of the first reaction vessel vertically downward, ensuring that the first cover and the first reaction vessel are sealed.
   At this time, for safety, the first groove is used to verify that the first reaction vessel and the first cover are properly aligned and maintained in their correct positions.
5) The rotation speed (stirring speed) of the first rotational shaft by the first driving motor of the first driving part may be set to 0~500rpm. In the case of a pig heart, the speed is set to 350 rpm, and the first mesh chamber is rotated to stir by setting 10 seconds in the clockwise direction, 8 seconds in the stop time, and 10 seconds in the counterclockwise direction.
6) At the same time, using the quantitative first transfer pump, the decellularization solution inside the first reaction vessel is circulated vertically, i.e., from the bottom to the top of the first reaction vessel, and at this time, the circulation speed of the first decellularization solution is set to, for example, 200ml/min.

Ultimately, although local decellularization progresses from the first xenogeneic organ tissue within the first mesh chamber, the first decellularization solution inside the first reaction vessel is well mixed by vertical circulation of the internal solution through the first transfer pump and stirring through rotation of the first mesh chamber, thereby obtaining a first decellularization solution with a homogeneous concentration in real time.

Accordingly, when sampling the first decellularization solution, it becomes easy to analyze the electrical conductivity, turbidity, absorbance (UV-Vis), etc. of the first decellularization solution, so that the progress of the decellularization process of the first decellularization solution may be accurately identified in real time.

7) FIG. 4 is a graph showing the turbidity analysis results of the first decellularization solution using ultrapure water (DW) during the decellularization process of each tissue of a pig heart (left atrium, right atrium, left ventricle, and right ventricle), and a graph showing the analysis of turbidity for each tissue over time during each decellularization process after performing a total of three ultrapure water processes [DW(1) - DW(2) - DW(3)].

As shown in Fig. 4(a), the increase in turbidity, i.e., the color of the first decellularization solution becoming turbid over time, indicates that the blood and impurities contained in the tissue are released from the tissue, and significant differences were observed between the tissues; for example, in the case of the left atrium, the turbidity was significantly higher than that of the right atrium.

Additionally, although the turbidity increased over time, a trend was observed in which the rate of increase gradually slowed.

When the DW(1) process is completed, all solution in the reactor is discharged as waste liquid, and two additional ultrapure water processes are performed as shown in Fig. 4(b). After the DW(3) process, the final turbidity analysis showed that the turbidity of all tissues was below 30 NTU, indicating that almost all impurities had been removed.

8) FIG. 5 is a graph showing the electrical conductivity analysis results of the first decellularization solution using ultrapure water (DW) during the decellularization process of each tissue of a pig heart (left atrium, right atrium, left ventricle, and right ventricle).

It is a graph analyzing the electrical conductivity of each tissue over time during the decellularization process after performing a total of three ultrapure water processes [DW(1) - DW(2) - DW(3)].

In FIG. 5(a), at the earliest time point of 3 minutes, a very high electrical conductivity was observed due to the first decellularization solution inside the first reaction vessel not being homogeneously mixed. However, as time progressed, the solution reached a stabilized state.

As shown in Fig. 5(b), when the DW(2)-DW(3) process was carried out, the electrical conductivity decreased significantly, similar to the turbidity, indicating that most impurities may be considered to have been removed.

9) One implication derived from the analysis results of turbidity and electrical conductivity for each heart muscle tissue is that that simply high turbidity does not necessarily mean high electrical conductivity.

For example, although the turbidity of the left atrium was the highest, the electrical conductivity was the lowest in the left atrium at 280 uS/cm, and the electrical conductivity of the left ventricle was significantly higher at about 670 uS/cm.

The correlation between turbidity and electrical conductivity is related to the first decellularization solution (substance) of the first xenogeneic organ tissue, so further related research is necessary.

10) FIG. 6 is a graph showing the results of analyzing the absorbance (UV-Vis) of the first decellularization solution of the left ventricle using ultrapure water (DW) during the decellularization process of each pig heart muscle tissue (left atrium, right atrium, left ventricle, and right ventricle), and the graph showing the analysis of the absorbance (UV-Vis) of each tissue over time during each decellularization process after performing a total of three ultrapure water processes [DW(1) - DW(2) - DW(3)].

FIG. 6(a1) shows the absorbance (UV-Vis) analysis results over time, and FIG. 6(a2) shows the area (integral value) of these peaks in a graph.

As with the analysis results of turbidity and electrical conductivity, the peak area increases over time, but the rate of increase tends to gradually slow down.

FIG. 6(b1) shows the plotting of the final data from each of the three ultrapure decellularization processes, while FIG. 6(b2) shows the peak area as a graph.

As shown in FIG. 6(b1), the absorbance (UV-Vis) results significantly decreased as the process progressed, which means it is gradually becoming similar to the intensity of the reference ultrapure water set as the baseline, proving that decellularization using ultrapure water was almost completed.

Results similar to the above were also obtained in tissues other than the left ventricle (left atrium, right atrium, right ventricle).

11) FIG. 7 shows the process of decellularization of heart muscle tissue using ultrapure water and various organic solvents. As it progresses from (a) to (b) to (c), it can be seen that the volume of the tissue decreases and its color changes to white.

(Test results for performance, etc.)

The most fundamental item when evaluating the quality of bioink of decellularized first xenogeneic organ tissue is the biochemical assay.

This quantitatively represents the content of DNA, collagen, and GAGs within the tissue.

[Table 1] shows the results of analyzing the DNA content of each tissue of the pig heart after decellularization.

Since the DNA content of each tissue is different, the general standard for judging that decellularization has been successfully completed is when the DNA content within the tissue is less than 50 ng/mg.

As shown in [Table 1], the DNA content of all tissues (left atrium, right atrium, left ventricle, and right ventricle) satisfied the above criteria, so it may be judged that decellularization was performed well.

**[Table 1]**

| left atrium | right atrium | left ventricle | right ventricle |
|---|---|---|---|
| 8.2 ng/mg | 6.7 ng/mg | 7.5 ng/mg | 6.2 ng/mg |

If DNA is released while Collagen and GAGs (glycosaminoglycans) are removed simultaneously, the physical properties of the bioink will not be good.

Therefore, it is necessary to analyze GAGs and collagen to confirm whether these two substances are well preserved within the tissue. As shown in [Table 2] and [Table 3], a large amount of GAGs and collagen was confirmed to be well preserved.

**[Table 2]**

| left atrium | right atrium | left ventricle | right ventricle |
|---|---|---|---|
| 1.7 ug/mg | 2.1 ug/mg | 1.6 ug/mg | 1.5 ug/mg |

**[Table 3]**

| left atrium | right atrium | left ventricle | right ventricle |
|---|---|---|---|
| 53.2 ug/mg | 63.1 ug/mg | 30.3 ug/mg | 30.1 ug/mg |

According to the above results, after decellularization of small quantities of various types of xenogeneic organs using the twin system of the first and second ex-situ analysis application devices, the DNA content of the left atrium, right atrium, left ventricle, and right ventricle tissues was reduced to an extremely low level, while collagen and GAGs within the tissues were very well preserved, indicating that high-quality bioink materials were effectively produced.

Although the present invention has been described through preferred embodiments as stated above, it is not limited thereto, and those skilled in the art to which the present invention pertains will easily understand that various modifications and variations are possible without departing from the scope of the claims described below.

### (Explanation of symbols)

100: first reaction vessel
200: second reaction vessel
RA1: first ex-situ analysis application device
RA2: second ex-situ analysis application device

## Claims

1. A decellularization reaction apparatus, comprises:
a first reaction vessel for storing a first decellularization solution to perform decellularization on the tissue of a first xenogeneic organ to be decellularized inside, and
one or more first ex-situ analysis application devices for extracting the first decellularization solution, which is undergoing a decellularization reaction with a tissue of the first xenogeneic organ designated for decellularization within the first reaction vessel, to the outside of the first reaction vessel, and for measuring a physical property of the first decellularization solution through ex-situ.

2. The decellularization reaction apparatus of claim 1, further comprises:
the first ex-situ analysis application device, and
a first mesh chamber that accommodates the tissue of the first xenogeneic organ designated for decellularization, which is inserted into the interior of the first reaction vessel.

3. The decellularization reaction apparatus of claim 2, wherein:
the first ex-situ analysis application device, comprises:
a first solution circulation part connected in a way that allows communication between the first reaction vessel and a first cover that covers the first reaction vessel, and intended to circulate a first decellularization solution to the first reaction vessel and the first cover, and
a first solution sampling part is connected to the first solution circulation part for extracting and sampling the first decellularization solution from the first solution circulation part to the outside.

4. The decellularization reaction apparatus of claim 3, comprises:
a first lifting part, which is combined with the first cover and allows the first cover to be lifted vertically.

5. The decellularization reaction apparatus of claim 4, wherein:
the first mesh chamber comprises a first driving part for rotating the first mesh chamber to be stirred within the first reaction vessel.

6. The decellularization reaction apparatus of claim 5, wherein:
the first mesh chamber is formed in a cylindrical shape with an empty interior.

7. The decellularization reaction apparatus of claim 6, wherein:
a outer surface of the first mesh chamber is arranged in a lattice form with a first mesh.

8. The decellularization reaction apparatus of claim 6, wherein:
a first lid for covering the first mesh chamber is detachably coupled to the upper part of the first mesh chamber.

9. The decellularization reaction apparatus of claim 1, wherein:
the first reaction vessel is manufactured from a transparent material or has a transparent window, enabling observation of the first reaction vessel interior from the outside.

10. The decellularization reaction apparatus of claim 9, wherein:
the first reaction vessel has a double jacket structure consisting of a first inner tube and a first outer tube.

11. The decellularization reaction apparatus of claim 10, wherein:
the first outer tube is equipped with a first refrigerant injection port for injecting refrigerant into the space between the first outer tube and the first inner tube, and a first refrigerant discharge port for discharging the injected refrigerant to the outside of the first outer tube.

12. The decellularization reaction apparatus of claim 8, wherein:
the first reaction vessel has a first solution outlet for discharging the decellularized solution outside of the first reaction vessel.

13. The decellularization reaction apparatus of claim 11, wherein:
the first cover is equipped with a first injection port for injecting the first decellularization solution into the interior of the first reaction vessel.

14. The decellularization reaction apparatus of claim 13, wherein:
the first cover is equipped with a first level sensor to measure the level of the first decellularization solution within the first reaction vessel.

15. The decellularization reaction apparatus of claim 5, wherein:
the first driving part, comprises:
a first rotational shaft coupled with a first lid of the first mesh chamber,
and
a first driving motor installed on the first cover of the first reaction vessel and coupled with the first rotational shaft to rotate the first rotational shaft.

16. The decellularization reaction apparatus of claim 12, wherein:
the first solution circulation part, comprises:
a first circulation tube connected between the first cover and the first solution outlet, for circulating the first decellularization solution within the first reaction vessel, and
a first transfer pump installed on the first circulation tube, to transfer the first decellularization solution discharged from the first solution outlet to the first circulation port.

17. The decellularization reaction apparatus of claim 16, wherein:
the first circulation tube is made of expandable a flexible tube.

18. The decellularization reaction apparatus of claim 17, wherein:
the first solution sampling part is connected to the first circulation tube and comprises a first solution sampling tube for extracting and sampling a first decellularization solution circulated in the first circulation tube.

19. The decellularization reaction apparatus of claim 18, wherein:
the physical property of the first decellularization solution are analyzed by measuring the absorbance (UV-Vis), electrical conductivity, and turbidity of the first decellularization solution sampled from the first solution sampling tube.

20. The decellularization reaction apparatus of claim 4, wherein:
the first lifting part, comprises:
a first coupling plate coupled to the first cover so as to be vertically movable, and
a first lifting cylinder having a first lifting rod, the first lifting cylinder being coupled to one end of the first coupling plate to raise and lower the first coupling plate.

21. A decellularization reaction apparatus, comprises:
a first reaction vessel for storing a first decellularization solution to perform decellularization on the tissue of a first xenogeneic organ to be decellularized inside,
one or more first ex-situ analysis application devices for extracting the first decellularization solution, which is undergoing a decellularization reaction with a tissue of the first xenogeneic organ designated for decellularization within the first reaction vessel, to the outside of the first reaction vessel, and for measuring a physical property of the first decellularization solution through ex-situ,
a second reaction vessel for storing a second decellularization solution to perform decellularization on the tissue of a second xenogeneic organ to be decellularized inside, and
one or more second ex-situ analysis application devices arranged parallel to the first ex-situ analysis application device and configured to extract the second decellularization solution, which is undergoing a decellularization reaction with the tissue of the second xenogeneic organ within the second reaction vessel, to the outside of the second reaction vessel and to measure a physical property of the second decellularization solution ex-situ.

22. The decellularization reaction apparatus of claim 21, wherein:
the second xenogeneic organ may be the same as or different from the first xenogeneic organ, and,
the second decellularization solution may be the same as or different from the first decellularization solution.

23. The decellularization reaction apparatus of claim 21, wherein:
the first ex-situ analysis application device, comprises:
a first mesh chamber that accommodates the tissue of the first xenogeneic organ designated for decellularization, which is inserted into the interior of the first reaction vessel,
a first solution circulation part connected in a way that allows communication between the first reaction vessel and a first cover that covers the first reaction vessel, and intended to circulate a first decellularization solution to the first reaction vessel and the first cover, and
a first solution sampling part is connected to the first solution circulation part for extracting and sampling the first decellularization solution from the first solution circulation part to the outside.

24. The decellularization reaction apparatus of claim 23, wherein:
the first ex-situ analysis application device, comprises:
a first lifting part, which is combined with the first cover and allows the first cover to be lifted vertically, and
the first mesh chamber comprises a first driving part for rotating the first mesh chamber to be stirred within the first reaction vessel.

25. The decellularization reaction apparatus of any one of claim 21 to claim 24, wherein:
the second ex-situ analysis application device, comprises:
a second mesh chamber that accommodates the tissue of the second xenogeneic organ designated for decellularization, which is inserted into the interior of the second reaction vessel.

26. The decellularization reaction apparatus of claim 25, wherein:
the second ex-situ analysis application device, comprises:
a second solution circulation part connected in a way that allows communication between the second reaction vessel and a second cover that covers the second reaction vessel, and intended to circulate a second decellularization solution to the second reaction vessel and the second cover,
and
a second solution sampling part is connected to the second solution circulation part for extracting and sampling the second decellularization solution from the second solution circulation part to the outside.

27. The decellularization reaction apparatus of claim 26, comprises:
a second lifting part, which is combined with the second cover and allows the second cover to be lifted vertically.

28. A decellularization reaction apparatus of claim 27, wherein:
the second mesh chamber comprises a second driving part for rotating the second mesh chamber to be stirred within the second reaction vessel.

29. The decellularization reaction apparatus of claim 28, wherein:
the second mesh chamber is formed in a cylindrical shape with an empty interior.

30. The decellularization reaction apparatus of claim 29, wherein:
a outer surface of the second mesh chamber is arranged in a lattice form with a second mesh.

31. The decellularization reaction apparatus of claim 30, wherein:
a second lid for covering the second mesh chamber is detachably coupled to the upper part of the second mesh chamber.

32. The decellularization reaction apparatus of claim 25, wherein:
the second reaction vessel is manufactured from a transparent material or has a transparent window, enabling observation of the second reaction vessel interior from the outside.

33. The decellularization reaction apparatus of claim 32, wherein:
the second reaction vessel has a double jacket structure consisting of a second inner tube and a second outer tube.

34. The decellularization reaction apparatus of claim 33, wherein:
the second outer tube is equipped with a second refrigerant injection port for injecting refrigerant into the space between the second outer tube and the second inner tube, and a second refrigerant discharge port for discharging the injected refrigerant to the outside of the second outer tube.

35. The decellularization reaction apparatus of claim 31, wherein:
the second reaction vessel has a second solution outlet for discharging the decellularized solution outside of the second reaction vessel.

36. The decellularization reaction apparatus of claim 35, wherein:
the second cover is equipped with a second injection port for injecting the second decellularization solution into the interior of the second reaction vessel.

37. The decellularization reaction apparatus of claim 28, wherein:
the first driving part, comprises:
a second rotational shaft coupled with a second lid of the second mesh chamber, and
a second driving motor installed on the second cover of the second reaction vessel and coupled with the second rotational shaft to rotate the second rotational shaft.

38. The decellularization reaction apparatus of claim 35, wherein:
the second solution circulation part, comprises:
a second circulation tube connected between a second cover and a second solution outlet, for circulating the second decellularization solution within a second reaction vessel, and
a second transfer pump installed on the second circulation tube, to transfer the second decellularization solution discharged from the second solution outlet to the second circulation port.

39. The decellularization reaction apparatus of claim 38, wherein:
the second circulation tube is made of expandable a flexible tube.

40. The decellularization reaction apparatus of claim 39, wherein:
the second solution sampling part is connected to the second circulation tube and comprises a second solution sampling tube for extracting and sampling a second decellularization solution circulated in the second circulation tube.

41. The decellularization reaction apparatus of claim 40, wherein:
the physical property of the second decellularization solution are analyzed by measuring the absorbance (UV-Vis), electrical conductivity, and turbidity of the second decellularization solution sampled from the second solution sampling tube.

42. The decellularization reaction apparatus of claim 27, wherein:
the second lifting part, comprises:
a second coupling plate coupled to the second cover so as to be vertically movable, and
a second lifting cylinder having a second lifting rod, the second lifting cylinder being coupled to one end of the second coupling plate to raise and lower the second coupling plate.
